# EUROPEAN PATENT APPLICATION

(11) **EP 0 982 397 A1**
(43) Date of publication of application: **01.03.2000**
(21) Application number: 98202181.8
(22) Date of filing: 29.06.1998
(51) Int. Cl.: C12N 9/64, A61K 38/38

(54) **Inhibition of contact system activation**

(71) Applicant: Stichting Sanquin Bloedvoorziening, 1066 CX Amsterdam (NL)
(72) Inventor: The designation of the inventor has not yet been filed
(74) Representative: Smulders, Theodorus A.H.J., Ir.

(57) **Abstract**

Novel inhibitors of the contact system were developed by a) the discovery that the sequence encompassing the amino acid residues 39 to 47 of the heavy chain region of human FXII contributes to the binding of FXII to activators such as negatively charged surfaces; and b) the demonstration that peptides mimicking this identified sequence inhibit the binding of FXII to activators and prevent contact system activation in human plasma. These novel contact system inhibitors may be applied to prevent activation of the contact system of blood by artificial surfaces, or used as anti-inflammatory drug for the prophylactic or therapeutic treatment of human or animal diseases.

## Description

### 1. Field of the Invention

This invention is in the field of medicine, in particular in prophylaxis and therapeutic treatment of animals, including human beings, having a condition which involves inflammation, coagulation, or both, and furthermore lies in the field of biochemistry.

More particularly, this invention relates to a novel class of inhibitors of the contact system of coagulation. Furthermore the invention relates to the use of these new inhibitors to prevent activation of the contact system of blood by artificial surfaces, or as anti-inflammatory drugs for the prophylactic or therapeutic treatment of human or animal diseases.

### 2. Background of the Invention

Blood clotting comprises a complex series of interactions of a number of plasma proteins, known as clotting factors. A central event of this clotting process is the conversion of the inactive prothrombin into the active serine-proteinase thrombin, which is the key enzyme catalyzing the conversion of the soluble protein fibrinogen into the insoluble fibrin. The proenzyme prothrombin is converted by a complex consisting of activated factors X (factor Xa) and V (factor Va), calcium ions and phospholipids. This complex may result from activation of two pathways, i.e. the extrinsic and the intrinsic pathway of activation.

The extrinsic pathway involves the membrane-bound protein tissue factor and the plasma protein factor VII. This extrinsic pathway is triggered by contact of blood with tissue factor exposed by cells or subendothelial structures. The intrinsic pathway involves the plasma proteins factor XII (FXII), prekallikrein, high molecular weight kininogen (HMWK), factor XI, factor IX and factor VIII. The precise role of the intrinsic pathway proteins in physiological hemostasis is still under investigation. Novel insights predict that factors VIII, IX and XI may amplify the generation of thrombin originating from extrinsic pathway activation, whereas the role of FXII, prekallikrein and HMWK is not well known. Genetic deficiencies of the latter proteins do not seem to be associated with bleeding disorders. Hence, FXII, prekallikrein and HMWK likely are not essential for normal hemostasis.

However, upon contact of blood with artificial surfaces, such as glass or biomaterials, these proteins may induce clotting. Because of this property FXII, prekallikrein and HMWK together are also known as the contact system of coagulation. There is ample evidence that contact system activation also contributes to inflammatory reactions, for example via the generation of bradykinin. Thus, inhibitors of contact system activation potentially can inhibit clotting of blood or reduce inflammatory reactions under certain pathophysiological conditions.

### 2.1 The contact system

The contact system of coagulation is that part of the clotting system that induces clotting upon contact of blood with artificial surfaces. Central in the activation of the contact system is the binding of the serine-proteinase factor XII (FXII; Hageman factor) to an activator followed by its activation. The proteins constituting the contact system (also known as the contact system of coagulation or the kallikreinkinin system) circulate in blood as inactive precursor proteins. Colman RW, 1984, J Clin Invest 73:1249; Kaplan AP et al., 1987, Blood 70:1; Kozin F, et al, 1992, In: Gallin JI, Goldstein IM, Snyderman R (eds): Inflammation: Basic Principles and Clinical Correlates, New York, Raven Press, p.103.

Contact system activation starts with the binding of FXII (formerly known as Hageman factor) to an activator. Bound FXII then becomes activated (FXIIa; the suffix "a" designates the active form of the protease), during which process it is converted from a single-chain inactive into a two-chain active serine-proteinase. Tans G et al., 1987, Sem Thromb Hemost 13:1. Upon binding, the conformation of FXII changes to yield a single chain species that, in contrast to native FXII, is highly susceptible to limited proteolysis by kallikrein. Griffin JH, 1978, Proc Natl Acad Sci USA 75:1998. Bound FXII is then activated by trace amounts of active kallikrein present in plasma. Kaplan, AP et al., 1987, Blood 70:1. An alternative hypothesis claims that FXII bound to an activator undergoes autoactivation. Dunn JT, et al., 1982, J Biol Chem 257:1779. FXIIa in its turn activates prekallikrein, that via its cofactor high molecular weight kininogen is bound to the activator, into the active serine-proteinase kallikrein. Wiggins RC, et al., 1977, Proc Natl Acad Sci USA 74:4636. Kallikrein in its turn activates bound but not yet activated FXII (reciprocal activation). FXIIa additionally can activate factor XI, which in turn activates factor IX to start activation of coagulation via the intrinsic pathway. Cochrane CG et al., 1982, Adv Immunol 33:290; Colman RW, 1984, J Clin Invest 73:1249; Kaplan AP et al., 1987, Blood 70:1; Kozin F et al, 1992, In: Gallin JI, Goldstein IM, Snyderman R (eds): Inflammation: Basic Principles and Clinical Correlates, New York, Raven Press, p.103.

### 2.2 Structure of factor XII

FXII is a serine-proteinase that is secreted by the liver as a single chain zymogen. The native protein has a molecular mass of 80 kDa. The normal plasma concentration is about 25-30 µg per ml. Both the primary structure of FXII as well as the nucleotide sequence of full-length cDNA have been reported. Fujikawa K et al., 1983, J Biol Chem 258:10924; McMullen BA, et al., 1985, J Biol Chem 260:5328. The mature protein consists of 596 amino acids, contains 20 disulfide bridges and presumably at least one carbohydrate group. Cool DE, et al., 1985, J Biol Chem 260:13666. Although discovered because of its procoagulant activity in vitro, the structure of FXII more resembles that of fibrinolytic proteins, in particular tissue-type plasminogen activator (tPA), rather than that of coagulation factors. Tans G et al., 1987, Sem Thromb Hemost 13:1; Cool DE, et al., 1985, J Biol Chem 260:13666. Several domains on FXII corresponding to the exon-intron organization of the gene have been identified. From the amino-terminal to the carboxy-terminal regions these domains are a fibronectin type-II domain, a growth factor-like domain, a fibronectin type-I domain, another growth factor-like domain, a kringle, a proline-rich region of undefined structure and a serine-proteinase catalytic region. Tans G et al., 1987, Sem Thromb Hemost 13:1; Cool DE, et al., 1985, J Biol Chem 260: 13666. A preliminary structural model for the serine-proteinase region has been proposed based on X-ray diffraction structures of trypsin, chymotrypsin and elastase. Until now, functional activities of FXII have not been assigned to these domains, except for the catalytic site and the site that mediates binding of FXII to activators. The latter has been localized on the amino-terminal 28 residues, more precisely on residues 5 to 15. Clarke BJ et al., 1989, J Biol Chem 264:11497. Although peptides corresponding to the amino-terminal sequence have been prepared, no functional studies with these or analogous peptides have been reported. Studies with recombinant FXII mutants that lack various heavy chain domains, suggest involvement of other residues in the binding of FXII to an activator. Citarella F, et al., 1992, Eur J Biochem 208:23; Citarella F, et al., 1996, Eur J Biochem 238: 240.

### 2.3 Biological effects of contact activation

The contact system often is activated in inflammatory conditions. Colman RW, 1984, J Clin Invest 73:1249; Kaplan AP et al, 1987, Blood 70:1; Kozin F, et al, 1992, In: Gallin JI, Goldstein IM, Snyderman R (eds): Inflammation: Basic Principles and Clinical Correlates, New York, Raven Press, p.103. Likely this activation amplifies inflammatory reactions, since during activation several biologically active fragments or proteases are formed such as bradykinin, kallikrein and activated FXII. These fragments or proteases may activate and degranulate neutrophils, increase vasopermeability and decrease vascular tonus. Colman RW, 1984, J Clin Invest 73:1249; Kozin F, et al, 1992, In: Gallin JI, Goldstein IM, Snyderman R (eds): Inflammation: Basic Principles and Clinical Correlates, New York, Raven Press, p.103.

Under pathological conditions, such as septic shock, the contact system may become activated and, via the generation of bradykinin, contribute to hypotension. Local activation of the contact system in the tissues may cause inflammation (via the effects of activated contact factors on neutrophils, macrophages and other cells) and enhance vasopermeability. Hence, inhibition of contact activation in human disease may help to reduce inflammation.

Persons with a genetic deficiency of FXII may have an increased risk for thromboembolic disease. This, together with its tPA-like structure (Tans G et al., 1987, Sem Thromb Hemost 13:1), suggests that FXII participates in fibrinolysis. In vivo observations on the contribution of FXII to plasminogen activation in homo- and heterozygous FXII deficient individuals are in agreement herewith. Levi M et al., 1991, J Clin Invest 88: 1155. The in vivo role of factor XI may be different: recent studies suggest that activation of factor XI may occur independently from FXII via thrombin and contribute to activation of factor IX. Naito K, et al., 1991, J Biol Chem 266:7353; Gailani D, et al., 1991, Science 253:909. In this view factor XI acts to amplify thrombin generation, initially induced by the extrinsic pathway. Davie EW et al., 1991, Biochemistry 30:10363; Broze Jr. GJ, 1992, Seminars Hematol 29:159. This supposed role of factor XI in the coagulation system is consistent with clinical data that the only deficiency of a contact system protein, which results in a (mild) bleeding disorder, is that of factor XI. Whatever its precise physiological role may be, there is, however, little doubt that FXII may become activated upon contact of blood with biomaterials, and that this activation triggers abnormal clotting and other unwanted activation processes, which limits the use of biomaterials.

Contact of blood with artificial surfaces may occur during collection and subsequent storage of blood or plasma of healthy donors, or during the fractionation process to prepare therapeutic protein preparations of plasma. Activation of factor XII may be induced by contact of the blood with collection bags, or during the process of fractionation, for example by contact with celite, used for filtration. Activated factor XII may induce activation (and consumption) of other clotting factors, such as factors VII and VIII, and induce the generation of the vasoactive nonapeptide bradykinin. Undesired activation of the contact system may also occur upon contact of blood with extra- or intracorporeal devices, for extracorporeal circuits used to bypass lung and heart during cardiac surgery. Thus, inhibition of contact activation may help to reduce unwanted side effects of biomaterials in vivo.

### 2.4 Proteinase inhibitors that regulate contact activation

Activation of the contact system is controlled by the serine-proteinase inhibitor (serpin) C1-inhibitor and by α2-macroglobulin. C1-inhibitor, a member of the superfamily of serpins (**ser**ine-**p**roteinase **in**hibitor**s**), inhibits activated C1, the first component of complement, FXIIa and kallikrein by forming stable complexes with these serine proteases. Schapira M et al., 1985, Complement 2:111; Davis AE, 1988, Ann Rev Immunol 6:595; Harpel P.C. et al., 1975, J Clin Invest 55:593. C1-inhibitor is also the major inhibitor of factor XIa. Wuillemin WA et al., 1995, Blood 85:1517. Thus, C1-inhibitor is not a specific inhibitor of FXIIa. Furthermore, under certain conditions, for example when bound to an activator, FXIIa is hardly inhibited by C1-inhibitor. Dors D et al., 1992, Thromb Haemost 67:644.

Alpha-2-macroglobulin is a multispecific proteinase inhibitor that inhibits proteinases of all four catalytic classes (serine-, cysteïne-, metallo-, and aspartate-proteinases). Barrett AJ et al., 1973, Bioch J 133:709; Travis J et al., 1983, Annu Rev Bioch 52:655. Among the proteinases inhibited by α2-macroglobulin is kallikrein. Harpel P, 1970, J Exp Med 132. In vivo, α2-macroglobulin probably acts as a second inhibitor of kallikrein (next to C1-Inh), whereas FXIIa is not inhibited by this inhibitor. As for C1-inhibitor the inhibition of the contact system by α2-macroglobulin is not specific.

Thus, although plasma contains a number of proteinase inhibitors including potent inhibitors of the contact system, these inhibitors are not specific and are less potent in inhibiting surface bound FXIIa.

### 2.5 Other inhibitors of factor XII

Various proteins, including C1q (a subcomponent of C1, the first component of complement), platelet factor 4, soluble human placental collagens types III, IV and V, and β2-glyco-protein I, are able to prevent binding of FXII to activators such as kaolin, ellagic acid and sulfatides. Henry ML et al., 1988, J Lab Clin Med 111:519; Dumenco LL et al., 1988, J Lab Clin Med 112:394; Rehmus EH et al., 1987, J Clin Invest 80:516; Koenig JM et al., 1991, J Lab Clin Med 117:523. In addition, peripheral blood mononuclear cells and endothelial cells contain substances, not yet defined, with similar properties. Ratnoff OD et al., 1991, Proc Natl Acad Sci USA 88:10740; Ratnoff OD et al., 1987, J Clin Invest 80:1180. These agents presumably have in common that they all have collagen-like regions via which they compete with FXII for binding to an activator. The potential use of these proteins as inhibitors of contact activation has not been explored.

### 3. Summary of the Invention

It has now been found that residues 39 to 47 of the heavy chain region of FXII, i.e. the sequence ³⁹YHKCTHKGR⁴⁷ (SEQ ID NO: 1), are involved in the binding of this serine-proteinase to activators such as artificial surfaces, and that peptides mimicking this sequence can be used to inhibit binding of FXII to, and its subsequent activation by, these surfaces. Therefore, the present invention contemplates the development of novel and selective inhibitors of FXII binding and activation. These inhibitors can be used prophylactically or therapeutically to inhibit contact system activation either triggered by contact of blood with artificial surfaces or biomaterials, or occurring in the pathogenesis of human or animal diseases.

The invention will be more fully understood after a consideration of the following description of the invention.

### 4. Brief Description of the Drawings

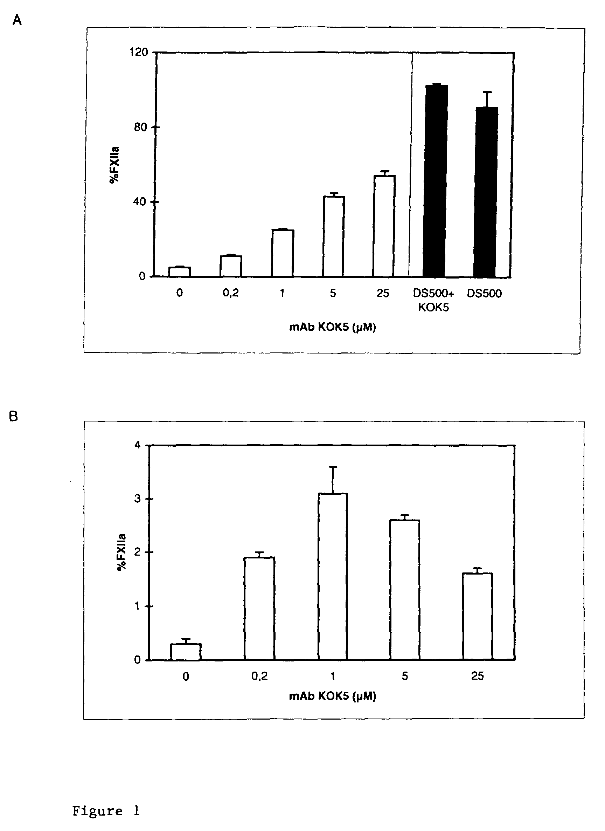

**Figure 1: Effects of mAb KOK5 on activation of purified FXII.**
   **A: Activation by kallikrein:** Twenty-five µl of prewarmed FXII (125 nM), 25 µl of serial dilutions of mAb KOK5, 25 µl of prewarmed kallikrein (25 nM) and 25 µl of buffer yielding final concentrations of 50 mM Tris-HCl, 150 mM NaCl, 0.1% (wt/vol) Tween-20, pH 7.8, were incubated for 30 min at 37°C. Thereafter, 50 µl of assay mixture containing the chromogenic substrate S-2302 were added and the increase in absorbance at 405 nm at 37°C was recorded. Results are expressed as percentage of the total amount of FXII present in the mixture (as determined by limited digestion with 10 nM trypsin), and represent the mean ± SE of two experiments. The activation in the presence of mAb KOK5 in the presence of dextran sulphate MW 500,000 (DS500; 50 µg/ml), as well as that by DS500 (50 µg/ml) alone is shown;
   **B: Autoactivation:** Twenty-five µl of prewarmed FXII (500 nM), 25 µl of serial dilutions of mAb KOK5 and 50 µl of buffer yielding final concentrations of 50mM Tris-HCl, 150mM NaCl, 0.1% (wt/vol) Tween-20, pH 7.8, were incubated for 60 min at 37°C. Thereafter, 50 µl of assay mixture containing S-2302 were added and the increase in absorbance at 405 nM at 37°C was recorded. Results represent the percentage of the total amount of FXII in the mixture, and represent the mean ± SE of two experiments.

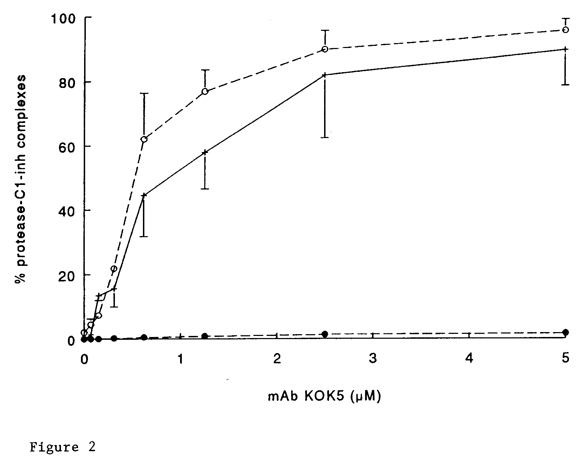

**Figure 2: Activation of contact system in plasma by mAb KOK5.**
   Forty ml of EDTA-plasma were incubated with 40 µl of two-fold dilutions of mAb KOK5 in PBS for 20 min at 37°C as described in Material and methods. The reaction was stopped by adding 120 µl of stop buffer (PBS, 0.1 mg/ml SBTI, Polybrene 0.05% wt/vol) and the amount of FXIIa-C1-inhibitor (+---+), kallikrein-C1-inhibitor (o- -o) and factor XIa-C1-inhibitor (●- -●) complexes generated was measured. Results, expressed as percentage of the maximum amount of complexes generated in pooled plasma by DS500 (50 µg/ml), represent the mean values ± S.D. from three different experiments.

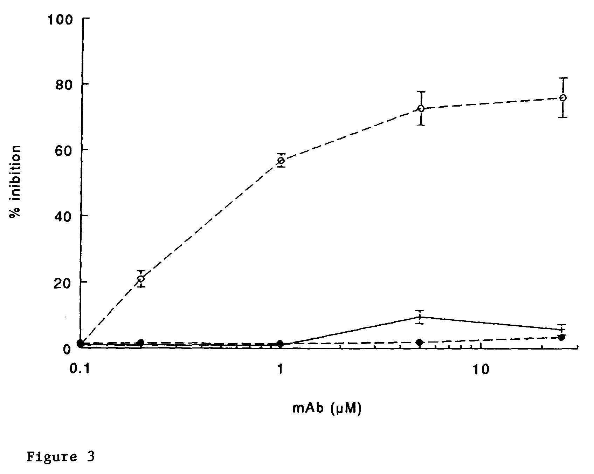

**Figure 3. Inhibition of FXII clotting activity by mAb KOK5.**
   Fifty µl of purified FXII (500nM) were preincubated with 50 µl of serial dilutions of either mAb KOK5 ( o- -o) or mAb F1 (●- -●) in PBS for 15 min and then tested in a FXII-clotting assay as described in Material and methods. In addition 50 µl of purified FXII (500 nM) were first incubated for 2 min with kaolin and then with serial dilutions of mAb KOK5 for 15 min (+---+); whereafter the residual FXII clotting activity was measured. Results are expressed as percentage of inhibition compared to the clotting activity measured in the absence of mAbs and are the mean ± SE of two experiments.

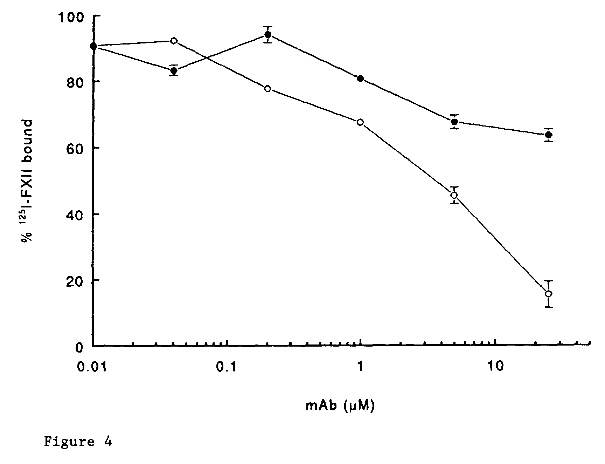

**Figure 4. Inhibition of FXII binding to kaolin by mAb KOK5.**
   Fifty µl of ¹²⁵I-FXII (0.027 pmoles/ml) were added to 0.5 ml polypropylene tubes and incubated at room temperature for 1 h with 10 µl of PBS, 0.1% (wt/vol) Tween-20 (PT) containing increasing amounts of mAbs (mAb KOK5 (o---o); mAb F1 (●---●)). Thereafter, 50 µl of kaolin (0.032 mg/ml in PT) were added. After 10 min at room temperature tubes were centrifuged for 2 min at 10,000 g and the pellets were counted for radioactivity. Binding was expressed as the percentage of the total counts that were found in the pellet in the absence of mAbs. Results represent the means ± SE of two experiments.

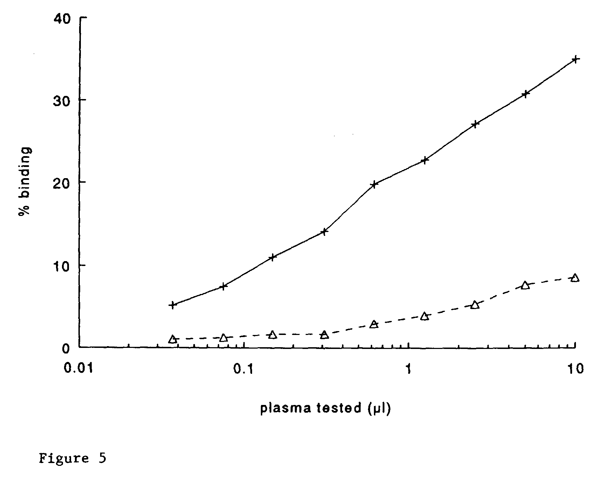

**Figure 5. Accessibility of the epitope for mAb KOK5 on different FXII species.**
   Two-fold serial dilutions of EDTA-plasma (△- -△) and DS-activated-plasma (+---+) were incubated with KOK5-Sepharose. Bound FXII was detected by subsequent incubation with ¹²⁵I-anti-FXII antibodies. Results are expressed as percentage of the ¹²⁵I-anti-FXII added and represent the means of two experiments.

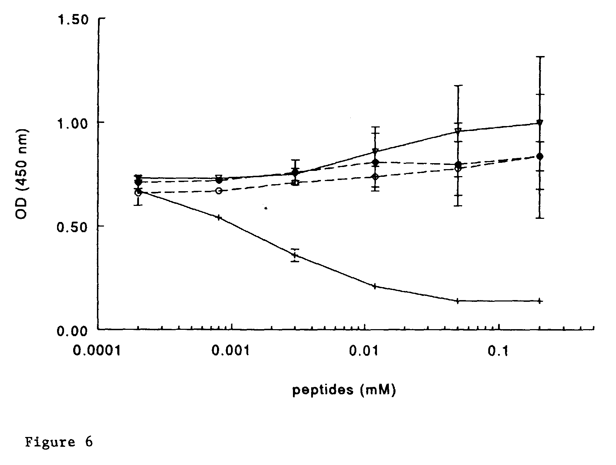

**Figure 6. Competition for binding of mAb KOK5 to factor XII by synthetic peptides.**
   Hundred µl of dilutions of the peptides were preincubated overnight at 4°C with 10 µl of biotinylated mAb KOK5 (10 µg/ml) in PT, 0.1% BSA. Thereafter samples were added to Maxisorp plates (96 wells) that had been coated overnight at 4°C with 100 µl of purified FXII (56 ng/ml) in 0.1 M NaHCO₃, pH 9.6, and blocked by incubation for 45 min at room temperature with washing buffer (PBS, 0.1% Tween 20 wt/vol). After 1 h incubation at room temperature plates were washed 5 times and then incubated for 30 min with streptavidin-horseradish peroxidase 1:1000 diluted in PTG. Bound peroxidase was visualized by incubating the plates with 0.11 M sodium acetate, pH 5.5, containing 0.1 mg/ml 3,3',5,5'-tetramethyl-benzidin and 0.003% (v/v) H₂O₂. Reaction was stopped with 2 M H₂SO₄ and absorbance was read at 450 nm. Results represent the means ± SE of two experiments.
   Symbols: AEGEFRFQTPQWE (+---+), SHQCTHRKCY (o- -o), ²⁹HFPFQYHRQ³⁷ (●- -●), ³⁹YHKCTHKGR⁴⁷ (∇---∇).

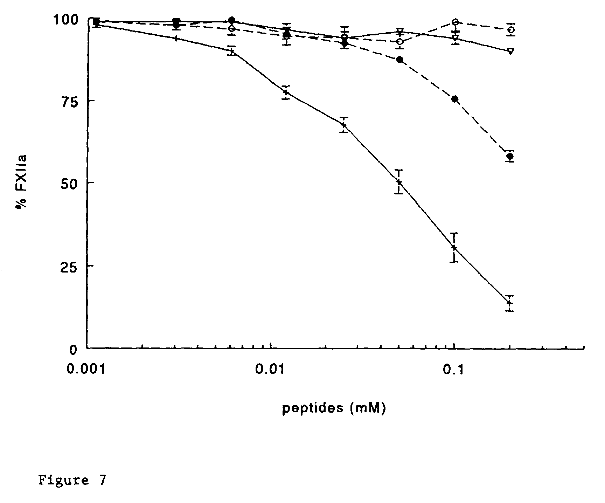

**Figure 7. Inhibition of purified FXII activation in the presence of mAb KOK5 by synthetic peptides.**
   Twenty-five µl of mAb KOK5 (1 mM) were preincubated for 30 mm at 37°C with 25 µl of serial dilutions of synthetic peptides, thereafter 25 µl of purified FXII (125 nM). The incubation buffer was 50 mM Tris-HCl, 150 mM NaCl, 0.1% (wt/vol) Tween-20, pH 7.8. After 15 min incubation at 37°C prewarmed kallikrein (25 nM) was added and the incubation was prolonged for 25 min. Thereafter, 50 µl of assay mixture containing S-2302 were added and the increase in absorbance at 405 nm at 37°C was recorded. Results are expressed as percentage of the total FXII activity determined by limited digestion with trypsin (10 nM), and represent the mean ± SE of two experiments.
   Symbols: AEGEFRFQTPQWE (+---+), SHQCTHRKCY (o- -o), ²⁹HFPFQYHRQ³⁷ (●- -●), ³⁹YHKCTHKGR⁴⁷ (∇---∇).

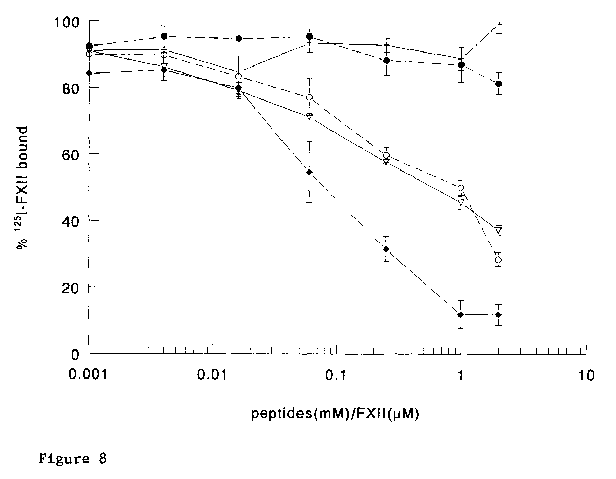

**Figure 8. Inhibition of FXII binding to kaolin by synthetic peptides.**
   Ten µl of serial dilutions of the peptides (or FXII) and 50 µl of ¹²⁵I-FXII (0.024 pmoles/ml) in PT were added to 50 µl of kaolin (0.032 mg/ml). After 10 min at room temperature, tubes were centrifuged and pellets were counted for radioactivity. Results are expressed as the percentage of the total counts that were found in the pellet in the absence of peptides and are the means ± SE of two determinations.
   Symbols: AEGEFRFQTPQWE (+---+), SHQCTHRKCY (o- -o), ²⁹HFPFQYHRQ³⁷ (●- -●), ³⁹YHKCTHKGR⁴⁷ (∇---∇), FXII (◆- -◆).

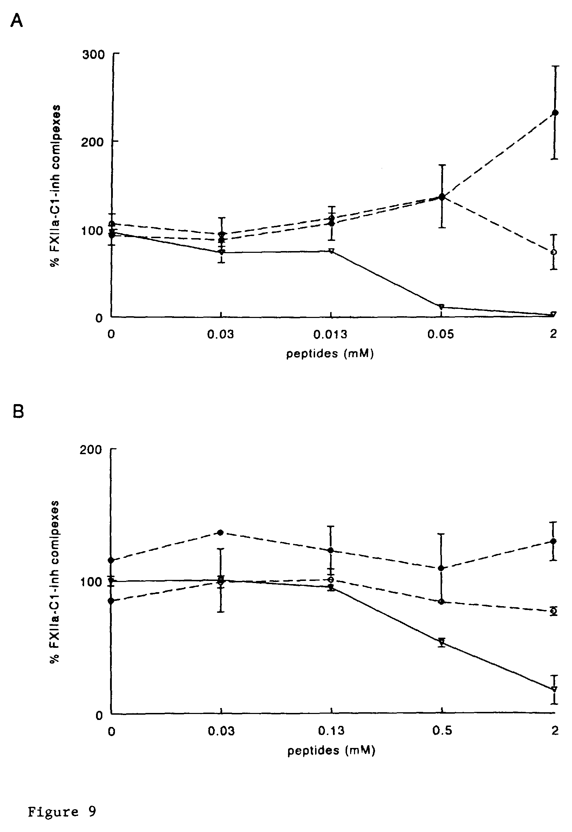

**Figure 9. Inhibition of kaolin induced contact system activation by synthetic peptides.**
   **A**: Twenty µl of DS500 (100 µl/ml in PBS) were preincubated for 10 min with 20 µl of serial dilutions of peptides, thereafter 40 µl of prewarmed EDTA-plasma were added. The microplate was shaken for 3 min at room temperature and then incubated for 20 min at 37°C, after which time the activation was stopped by adding 120 µl of stop buffer (see materials and methods). The amount of FXIIa-C1-inh complexes was determined. Results are expressed as the percentage of the maximum amount of each complex generated by DS500 in the absence of peptides and are the means ± SE of two determinations.
   **B**: Similarly, 20 µl of kaolin (5mg/ml) were mixed with 20 µl of serial dilutions of peptides and 40 µl of EDTA-plasma. After 20 min incubation at 37°C the reaction was stopped by adding 120 µl of stop buffer, and the amount of FXIIa-C1-inh complexes generated was measured. Results are expressed as the percentage of the maximum amount of each complex generated by kaolin in the absence of peptides and are the means ± SE of two determinations.
      Symbols: SHQCTHRKCY (o- -o), ²⁹HFPFQYHRQ³⁷ (●- -●), ³⁹YHKCTHKGR⁴⁷ (∇---∇).

### 5. Detailed Description of the Invention

Several patents/patents applications and scientific articles are referred to below that discuss various aspects of the materials and methods used to realize the invention. It is intended that all of the references be entirely incorporated by reference.

The kernel or gist of the present invention is the discovery that the sequence ³⁹YHKCTHKGR⁴⁷ (SEQ ID NO: 1) of the heavy chain region of FXII contributes to the binding of FXII to and its subsequent activation by an activator. Hence, peptides or drugs homologous to or mimicking this sequence (i.e. comprising a functionally equivalent sequence) can be used to block contact activation.

Activation of the contact system by artificial surfaces is initiated by binding of factor XII to these surfaces. Now that we have identified the amino acid sequence of that part of the factor XII molecule which mediates the binding of factor XII to a surface, peptides having or comprising this sequence can be made that will similarly bind to the surfaces and thereby will prevent the binding and subsequent activation of factor XII.

Such peptides could, e.g., be dissolved at an appropriate concentration in a buffer (such as physiological saline) and then be flushed through an extracorporeal circuit or, more generally, be contacted with artificial surfaces that may be exposed to contact with blood. The peptides will then bind to potential binding sites for factor XII and prevent binding of factor XII during subsequent contact of the circuit or surfaces with blood. Alternatively, the peptides could be linked, e.g., covalently, to surfaces that may become exposed to contact with blood, either directly or via a suitable spacer molecule.

Peptides having or comprising an amino acid sequence identical or homologous to that of the binding site on factor XII for activators, could also be used for the treatment of animals, including human beings, suffering from a disease which involves unwanted contact activation. The peptides could, e.g., be dissolved in a pharmaceutically acceptable vehicle and then be administered to an animal or human individual in need of treatment, for example by intravenous injection. The peptides will then compete with endogenous factor XII for the binding sites on the activator. To slow down clearance of the peptides it may be desirable to link the peptides to a carrier molecule, such as for example albumin.

It is considered to be well within the reach of the person skilled in the art to choose alternative administration routes, such as intra-arterial, intramuscular, subcutaneous injections, intravenous infusions; to choose suitable carriers or diluents and prepare suitable pharmaceutical compositions containing a peptide of the invention and such pharmaceutically acceptable carrier or diluent; to determine optimal doses and regimes of administration, depending on various factors such as the age, sexe, weight and condition of the animal/individual to be treated, the severity of the specific condition to be treated, etc.; to prepare a complex of a peptide of the invention and a suitable carrier molecule, such as albumin; etc.

Peptides of the invention may consist of the nonapeptide ³⁹YHKCTHKGR⁴⁷ (SEQ ID NO: 1) or a homologue thereof, which is essentially a similar sequence having a similar capability of binding to Factor XII activators, such as artificial surfaces.

Furthermore, peptides of the invention may be larger than such a nonapeptide. Such larger peptides should comprise the sequence ³⁹YHKCTHKGR⁴⁷ (SEQ ID NO: 1), but do not encompass the complete Factor XII molecule. In a preferred embodiment, the fibronectin type II homology region of Factor XII is used as a peptide of the invention. Said fibronectin type II homology region consists of amino acid residues 13-69 of Factor XII. The amino acid sequence of said region is: ¹³KAEEHTVVLTVTGEPCHFPFQY HRQLYHKCTHKGRPGPQPWCATTPNFDQDQRWGYC⁶⁹ (SEQ ID NO: 2). Any peptide derived from this fibronectin type II homology region and comprising SEQ ID NO: 1, or a homologue thereof, qualifies as a suitable peptide of the invention. For example, a preferred peptide of the invention is the peptide: ¹⁶EHTVVLTVT GEPCHFPFQYHRQLYHKCTHKGRPGPQPWCATTPNFDQDQRWGYC⁶⁹ (SEQ ID NO: 3).

The peptide of the invention may comprise a part which is not derived from Factor XII. Such part could be, or comprise, an extension of amino acid residues having another function, for example to link the Factor XII derived part of the peptide of the invention to a carrier molecule, e.g., albumin and other proteins, or to link the Factor XII derived part of the peptide to an active agent of choice. The peptide may comprise a part which retards its degradation by blood components, such as proteolytic enzymes. As an example of such a part, the peptide may include one or more D-amino acid residues.

To more clearly define the present invention, it will be described in the next sections. First, the materials and methods used are described; then the experiments leading to the identification of residues 39 to 47 as being involved in the binding of FXII to an activator, and finally an application of the discovery, i.e. the use of peptides carrying the identified sequence to inhibit contact activation in human plasma.

### 5.1 Material and methods

### 5.1.1 General

Dextran sulfate of nominal Mr 500,000 (DS500) and soybean trypsin inhibitor (SBTI) were from Sigma Chemical Co. (St. Louis, MO, USA). Hexadimethrine bromide (Polybrene) was from Janssen Chimica (Beerse, Belgium), the chromogenic substrate H-D-Pro-Phe-Arg-p-nitroanilide (S-2302) was from Chromogenix AB (Mölndal, Sweden), and CNBr-activated Sepharose 4B, Protein G-Sepharose and benzamidine-Sepharose from Pharmacia Fine Chemicals AB (Uppsala, Sweden). Plasma deficient for FXII, prekallikrein or high molecular weight kininogen were obtained from George King Biomedical Inc. (Overland Park, KS).

### 5.1.2 Proteins

FXII was immunopurified from 150 ml of human citrated plasma using a 20 ml column of mAb F3-Sepharose as described. Nuijens JH et al., 1989, J Biol Chem 264:12941. Thereafter the preparation was applied onto a benzamidine-Sepharose column to remove activated FXII. The amount of activated FXII present in the final preparation was 0.3% of the total amount of protein as determined by amidolytic activity towards the chromogenic substrate S-2302.

Prekallikrein was immunopurified from fresh human citrated plasma using a column of mAb K15-Sepharose as described. Ravon DM et al., 1995, Blood 86:4134.

Kallikrein was prepared by incubating prekallikrein with β-FXIIa coupled to Sepharose 4B (molar ratio of β-FXIIa to prekallikrein was 1 to 100) for 2 hours at 37°C, after which β-FXIIa-Sepharose was removed by centrifugation. SDS-PAGE analysis showed complete conversion of prekallikrein into kallikrein.

Protein concentrations were determined by radio-immunoassay. Nuijens JH et al., 1988, Blood 72:1841.

Purified FXII was labeled with ¹²⁵I by the chloramine-T method. Free labeled was separated by dialysis against phosphate buffered saline, pH 7.4 (PBS) containing 0.1% (wt/vol) Tween-20. The specific activity of the labeled preparation was 4.2 x 10⁷ cpm/mg of protein. The labeled FXII was electrophoresed on SDS/polyacrylamide gel and visualized by auto-radiography to assess the quality of preparation.

### 5.1.3 Monoclonal antibodies

MAb KOK5, an IgG1 antibody, was obtained by fusing spleen cells from mice immunized with 27 µg of purified human FXII with Sp 2/0-Ag14 myeloma cells, according to a procedure previously described. Nuijens JH et al., 1989, J Biol Chem 264: 12941. Culture supernatants were screened for the presence of specific antibodies by an enzyme-linked immunoassorbent assay, in which purified FXII was used as antigen. Nuijens JH et al., 1989, J Biol Chem 264:12941. Positive clones were subcloned by limiting dilution. MAb KOK5 was precipitated from hybridoma conditioned medium by 50% (wt/vol) ammonium sulphate, extensively dialysed against PBS, and affinity purified on Protein G-Sepharose according to manufacturer's instructions. F(ab')₂ and F(ab') fragments of the mAb KOK5 were prepared as described. MAbs F1 and F3 directed against an epitope localized on the kringle domain and on the light-chain region of FXII, respectively, have been described before. Nuijens JH et al., 1989, J Biol Chem 264:12941; Rayon DM et al., 1995, Blood 86: 4134.

MAbs were coupled to CNBr-activated Sepharose according to manufacturer's instructions. The beads were suspended in PBS containing 0.1% (wt/vol) Tween-20, 10 mM EDTA, 0.05% (wt/vol) Polybrene, 0.02% (wt/vol) NaN₃, and 0.01% (wt/vol) SBTI, which buffer efficiently inhibits activation of FXII during the incubation procedure. For biotinylation or radiolabeling with ¹²⁵I by the iodogen method mAbs were affinity purified on Protein G-Sepharose according to manufacturer's instructions.

### 5.1.4 FXII activation by kallikrein

Activation of FXII by kallikrein was carried out in a 96-well microtiter plate (Nunc, Roskilde, Denmark): 25 µl of pre-warmed FXII (125 nM), 25 µl of two-fold dilutions of mAb KOK5, its F(ab')₂ or F(ab') fragments, 25 µl of prewarmed kallikrein (25 nM) and 25 µl of buffer to yield final concentrations of 50 mM Tris-HCl, 150 mM NaCl, 0.1% (wt/vol) Tween-20, pH 7.8 in the mixture, were incubated for 30 min at 37°C. The amount of FXIIa formed was determined from the rate of hydrolysis of the chromogenic substrate S-2302: 50 µl of assay mixture (S-2302 1 mM, SBTI 100 mg/ml in Tris 50 mM, NaCl 150 mM, pH 7.8) were added to the wells and the increase in absorbance at 405 nm was recorded at time intervals of 2 min by a Bio-Kinetics Reader (Bio-tek instruments Inc.) set at 37°C. Under these conditions, the rates of increase in absorbance were constant for at least 10 min and were used to calculate the amount of FXIIa present in the wells on the basis of a calibration curve obtained with known amounts of fully activated FXII. Results are expressed as percentage of the total amount of FXII present in the mixture. In each experiment this amount was assessed by activating FXII with trypsin under limited proteolytic conditions. Variation of FXIIa generated under these conditions was less than 10% in all experiments and agreed well with the concentration of FXII present in the wells. In control experiments, it was established that the amount of SBTI added was sufficient to prevent conversion of the chromogenic substrate by kallikrein up to a concentration of 100 nM.

### 5.1.5 FXII autoactivation

Autoactivation of FXII was studied in a 96-well microtiter plate: 25 µl of FXII (500 nM), 25 µl of 2-fold serial dilutions of mAb KOK5 and 50 µl of buffer yielding final concentrations of 50 mM Tris-HCl, 50 mM NaCl, 0.1% (wt/vol) Tween-20, pH 7.8, were incubated for 60 min at 37°C. Thereafter, 50 µl of assay mixture (see above) were added to wells and the increase in absorbance at 405 nm was recorded at time intervals of 2 min. Under these conditions, the rates of increase in absorbance were constant for at least 10 min and were used to calculate the amount of FXIIa present in the wells as described above.

### 5.1.6 Activation of contact system in plasma

Pooled normal human plasma containing 10 mM EDTA (EDTA-plasma) was obtained as described. Nuijens JH et al., 1988, Blood 72:1841. Plasma activation was carried out in 96 wells microplates Dynatech (Plochingen, Germany): 40 µl of EDTA-plasma were preincubated for 5 min at 37°C, thereafter 40 µl of prewarmed serial dilutions of mAb KOK5 in PBS were added. The microplate was shaken for 3 min at room temperature and then incubated for 20 min at 37°C, after which time the activation was stopped by adding 120 µl of stop buffer (PBS, 0.1 mg/ml SBTI, Polybrene 0.05%, wt/vol).

To study the effects of synthetic peptides on the activation of contact system in EDTA-plasma in the presence of DS500, 20 µl of DS500 (100 µg/ml in PBS) were preincubated for 10 min with 20 µl of serial dilutions of peptides, thereafter 40 µl of prewarmed EDTA-plasma were added. The microplate was shaken for 3 min at room temperature and then incubated for 20 min at 37°C, after which time the activation was stopped by adding 120 µl of stop buffer (see above).

To study the effects of synthetic peptides on the activation of contact system in EDTA-plasma by kaolin, 20 µl of kaolin (5mg/ml) were mixed with 20 µl of serial dilutions of peptides and 40 µl of EDTA-plasma. After 20 min incubation at 37°C the reaction was stopped by adding 120 µl of stop buffer.

### 5.1.7 Assays for contact system activation in plasma

Contact system activation in plasma was assessed by measuring the generation of FXIIa-C1-inhibitor, kallikrein-C1-inhibitor and factor XIa-C1-inhibitor complexes. The amount of FXIIa-C1-inhibitor and kallikrein-C1-inhibitor complexes generated in EDTA-plasma was determined by radio-immunoassays, as described. Nuijens JH et al., 1988, Blood 72:1841. Sample dilutions were tested in duplicate and results were calculated by comparison with an in-house standard that consisted of normal pooled human plasma fully activated by DS500 (50 µg/ml). Generation of factor XIa-C1-inhibitor complexes was determined by an enzyme-linked immunoassorbent assay (ELISA) using kaolin-activated reference plasma. Wuillemin WA et al., 1995, Blood 85:1517.

### 5.1.8 Clotting assay for FXII

FXII-specific coagulant activity was determined by a modification of the activated partial thromboplastin time using FXII-deficient plasma. Fifty µl of purified FXII (0.5 µM) were preincubated for 15 min with 50 µl of serial dilutions of mAb KOK5, its F(ab')₂ or F(ab') fragments in PBS. Thereafter three different dilutions of the mixtures (50 µl) were mixed with 50 µl of FXII-deficient plasma and 50 µl of a mixture of phospholipids and 0.25% (wt/vol) kaolin in 20 mM Tris-HCl, pH 7.2. After an incubation of 4 min at 37°C, 50 µl of 25 mM CaCl₂ were added and the clotting time was measured with a coagulation timer (LAbor Fibrin timer, COA DATA 1000, Manufactures LAbor GmbH, Hamburg). The clotting activity of the samples was calculated on the basis of a calibration curve obtained with pooled normal human plasma. Results were expressed as percentage of inhibition compared to the activity measured in the absence of mAb assumed to be 100%.

### 5.1.8 Binding to kaolin

Binding of labeled FXII to kaolin was determined as described with minor modifications. Kunapuli SP et al., 1993, J Biol Chem 268:2486. Briefly 50 µl of ¹²⁵I-FXII (0.025 pmoles/ml) were added to 0.5 ml polypropylene tubes and incubated at room temperature for 1 h with 10 µl of PBS, 0.1% (wt/vol) Tween-20 (PT) containing increasing amounts of mAbs. Thereafter, 50 µl of kaolin (0.032 mg/ml in PT) were added and the mixture was incubated for 10 min at room temperature. The tubes were centrifuged for 2 min at 10,000 g and the pellets were counted for radioactivity. Binding was expressed as the percentage of the total counts that were found in the pellet in the absence of mAbs. Under these conditions, no significant binding of ¹²⁵I-FXII to the tubes (less than 0.5%) was observed. To study the effect of synthetic peptides on the binding of ¹²⁵I-FXII to kaolin, 10 µl of serial dilutions of the peptides and 50 µl of ¹²⁵I-FXII (0.025 pmoles/ml) in PT were added to 50 µl of kaolin (0.032 mg/ml). After 10 min at room temperature, tubes were centrifuged and pellets were counted for radioactivity. Binding was expressed as the percentage of the total counts that were found in the pellet in the absence of peptides.

### 5.1.9 Identification of the epitope recognized by mAb KOK5

Two random phage-displayed libraries were used in this study: A 15-mer peptide library and a 9-mer peptide library fused to pVIII. Devlin JJ et al., 1990, Science 249:404; Felici F et al., 1991, J Mol Biol 222:301. The affinity purification of phages bearing peptides recognized by the mAb KOK5 was performed as follows: 7.5 µg of mAb KOK5 were bound to 3 mg of Dynatech beads coated with anti-mouse IgG antibodies (Dynal A.S, Oslo, Norway), according to manufacturers procedure. Thereafter 1 x 10¹² phage (50 µl) from the twice amplified 15-mer library were incubated with 6 µg of KOK5-beads in 200 µl of PBS, 0.1% (wt/vol) BSA at 4°C for 16 h on the head over head rotation. Unbound phages were discarded and beads were washed 10 times with 1 ml PBS, 0.1% (wt/vol) Tween-20, 0.1% (wt/vol) BSA, thereafter bound phages were eluted with 200 µl of 0.1 M HCl, adjusted to pH 2.2 with glycine, for 15 min at room temperature. The eluate was transferred to a microfuge tube, neutralised with 12 µl of 2 M Tris base and amplified as described. Luzzago A et al., 1993, Gene 128:51. It was then used for a second round of panning. A control reaction and panning were carried out on beads coated with an irrelevant mAb. A third round of panning was carried out using ten times less immobilized mAb. Reactive clones were identified from the output of the third round of selection by an ELISA using a polyclonal sheep-anti-M13 biotin conjugate (5 Prime-3 Prime, Boulder, CO, USA). Briefly, 100 µl of mAb KOK5 (2.5 µg/ml in 0.1 M NaHCO₃ pH 9.6) were added to microtiter plates (Nunc, Roskilde, Denmark) and incubated overnight at 4°C. Individual clones were grown overnight and centrifuged for 5 min at 10,000 g. After blocking the wells of the microtiter plates with 2% milk in PBS, 100 µl of culture supernatant containing phage particles were added to each well and incubated for 2 h. After washes, wells were incubated with 100 µl of biotinylated polyclonal antibody against M13 phage 1 to 1500 diluted in PBS, 0.1% (wt/vol) Tween-20, 0.2% (wt/vol) gelatin (PTG) for 1 h. Thereafter, wells were washed and incubated for 30 minutes with streptavidin-horseradish peroxidase (Amersham, Amersham, UK), 1 to 1000 diluted in PTG. Bound peroxidase was visualized by incubating the plates with 0.11 M sodium acetate, pH 5.5, containing 0.1 mg/ml 3,3',5,5'-tetramethyl-benzidin (Merck, Darmstadt, Germany) and 0.003% (v/v) H₂O₂. Reaction was stopped with 2 M H₂SO₄ and absorbance was read at 450 nm on a Multiskan plate reader (Labsystems, Helsinki, Finland). More than 80% of clones were found to give a positive signal and 18 were chosen to be sequenced.

The nucleotide sequence of the DNA inserted in the selected clones was determined on the DNA fragments obtained by polymerase chain reaction (PCR) using the appropriate primers. Devlin JJ et al., 1990, Science 249:404. PCR products were purified by QIAquick PCR purification kit (QIAGEN GmbH, Hilden, Germany) and sequenced using Taq Dye Deoxy^{™} Terminator Cycle Sequencing Kit (Amersham, Buckinghamshire, UK) and the Applied Biosystems Model 373A DNA Sequencing System. Phages binding to mAb KOK5 were isolated from the 9-mer peptide library by a similar procedure as described above for 15-mer peptides. After the third round of panning positive plaques were identified by immunoscreening on nitrocellulose filters as described. Luzzago A et al., 1993, Gene 128:51. The isolated plaques were then grown to be tested in the ELISA and to be sequenced. Twenty-three clones were sequenced using the DYEnamic™ Direct cycle sequencing kit (-40 M13 DYEnamic ET primer) (Amersham, Buckinghamshire, UK) following manufacturer's instruction. Amino acid sequences deduced from nucleotide sequences are given in the single letter code and were aligned to FXII sequence by using PC/Gene software release 6.60 July 1,1991 (IntelliGenetics Inc.).

### 5.1.10 Peptides

Synthetic peptides were synthesized at the synthetic peptide facility of the Amsterdam-Leiden Institute for Immunology by Dr JW Drijfhout (Dept. of Immunohaematology and Blood Bank, University Hospital Leiden, Leiden). The peptides were resuspended in dimethyl sulphoxide at an approximate concentration of 10 mM and stored at -70°C.

### 5.2 Results

Essential for the present discovery was the identification of a monoclonal antibody (mAb KOK5) that inhibits both the clotting activity of FXII, as well as the binding of FXII to activators such as kaolin. Using peptide phage libraries the epitope on FXII for mAb KOK5 was identified and the functional activities of peptides mimicking the sequence of the epitope recognized by mAb KOK5 was assessed.

### 5.2.1 Effects of mAb KOK5 on the activation of purified FXII

Upon binding to an activator FXII undergoes conformational changes that lead to an enhanced susceptibility for kallikrein cleavage. In initial experiments we established that mAb KOK5 had a comparable effect. Activation of purified FXII by kallikrein was dose-dependently enhanced by mAb KOK5. After 30 min of incubation maximal activation was observed at a mAb:FXII molar ratio of 40:1 (Fig. 1A). The amount of FXIIa formed was 60% of the total amount of FXII present. If DS500 (50 µg/ml) was added, activation by kallikrein in the presence of mAb KOK5 generated 90% of FXIIa whereas, in the absence of any activator, only 5% of total FXII was formed (Fig. 1A). In separate experiments mAb KOK5 was demonstrated not to have any effect on the amidolytic activity of FXIIa. Together these experiments showed that mAb KOK5 could substitute for DS500 regarding activation of FXII by kallikrein.

The presence of mAb KOK5 also supported FXII auto-activation in a dose-dependent fashion reaching an optimum at a mAb:FXII molar ratio 1:0.8 and decreasing at higher concentrations (Fig. 1B). At optimal conditions almost 4% of FXII was activated after 1 hours incubation at 37°C in the presence of mAb KOK5, 61% in the presence of DS500 (2 µg/ml) and 0.3% in the absence of any activator.

### 5.2.2 Activation of the contact system in plasma by mAb KOK5

As it enhanced FXII activation by kallikrein and supported FXII autoactivation, mAb KOK5 was investigated for its potency to induce contact system activation in plasma. To this, normal human EDTA-plasma was incubated with increasing amounts of mAb KOK5 and the activation of contact system was assessed by measuring the generation of FXIIa-, kallikrein- and factor XIa-C1-inhibitor complexes generated. Results were expressed as the percentage of the maximum amount of each complex generated in normal human EDTA-plasma by DS500, which was arbitrarily set at 100. As shown in Fig. 2, protease-C1-inhibitor complexes were generated by mAb KOK5 in plasma in a dose-dependent manner. The maximum amount of FXIIa-C1-inhibitor and kallikrein-C1-inhibitor complexes was generated at a mAb:FXII molar ratio of 10 : 1 and was comparable to that obtained by activating the contact system with DS500. In contrast, the amount of factor XIa-C1-inhibitor complexes formed when plasma was incubated with mAb KOK5 was less than 2% of the amount formed in the presence of DS500. No protease-C1-inhibitor complexes were formed when mAb KOK5 was added to factor XII-, kallikrein- or high molecular weight kininogen-deficient plasma (data not shown). Activation of contact system was also observed when F(ab')₂ or F(ab') fragments of mAb KOK5 were added to EDTA-plasma, though somewhat less complexes were generated than with intact mAb KOK5 (Table 1). Polybrene (0.1%) only slightly decreased the activation of contact system by mAb KOK5. No activation was induced by the mAb F3 used as control (Table 1). Together these results indicated that mAb KOK5 induced activation of FXII and prekallikrein, but not that of FXI, in plasma.

**Table 1**

| Contact system activation in plasma induced by mAb KOK5 | | |
|---|---|---|
| Activator | FXIIa-C1-inhibitor | kallikrein-C1-inhibitor |
| mAb KOK5 | 81.9 ± 19.4 | 89.9 ± 5.8 |
| F(ab')₂KOK5 | 77.2 ± 34.3 | 75.6 ± 6.6 |
| F(ab') KOK5 | 20.3 ± 7.3 | 40.8 ± 6.5 |
| KOK5+Polybrene | 79.2 ± 13.4 | 54.1 ± 12.2 |
| mAb F3 | 0.3 | 0.5 |
| Forty µl of EDTA-plasma were incubated, for 20 min at 37°C, with 40 µl of mAb KOK5 (2.5 µM) with or without Polybrene (0.05% (wt/vol)), KOK5 (Fab')₂ (2.5 µM), KOK5 (Fab') (5 µM) and mAb F3 (2.5 µM) as control. The reaction was stopped by adding 120 µl of stop buffer (PBS, 0.1 mg/ml SBTI, Polybrene 0.05%, wt/vol). The amount of FXIIa-C1-inhibitor and kallikrein-C1-inhibitor complexes generated was measured as described in Material and Methods. Results, expressed as percentage of the maximum amount of complexes generated in pooled plasma by DS500 (50 g/ml), represent the mean values ± S.D. from three different experiments. | | |

### 5.2.3 Effects of mAb KOK5 on FXII clotting activity

Next the effect of the mAb KOK5 on FXII clotting activity was investigated. MAb KOK5 was found to inhibit clotting activity of purified FXII in a concentration dependent manner. Maximal inhibition was 73.5% of the total coagulant activity (Fig. 3) and was observed at a 10:1 molar ratio of intact mAb to FXII. F(ab')₂ and F(ab') fragments of mAb KOK5 also inhibited FXII clotting activity, to 63.1% and 60.5% respectively, at molar ratio of 20:1 (Fab')₂ and 40:1 (Fab'). MAb F1 used as a control did not have any effect on FXII clotting activity (Fig. 3). To evaluate the mechanism by which mAb KOK5 inhibited FXII clotting activity, we studied the capability of the mAb KOK5 to inhibit the clotting activity of kaolin-bound purified FXII. As shown in Fig. 3, mAb KOK5 was not able to inhibit the clotting activity of purified FXII preincubated with kaolin at any concentration tested.

### 5.2.4 Effect of mAb on the binding of FXII to kaolin

A possible explanation for the effects of mAb KOK5 on FXII clotting activity was that it interfered with the binding of FXII to negatively charged surfaces. Therefore, we analyzed the binding of ¹²⁵I-FXII to kaolin in the presence of mAb KOK5. MAb KOK5 inhibited binding of FXII to kaolin in a dose dependent manner whereas no inhibition was observed by other mAbs such as mAb F1 (shown in Fig. 4) or mAb F3.

### 5.2.5 Accessibility (exposure) of the epitope for mAb KOK5 on different FXII species

To analyze whether the epitope for mAb KOK5 was preferentially exposed on activated FXII, KOK5-coupled Sepharose beads were incubated with EDTA-plasma, containing native FXII, or with DS-activated plasma, containing activated FXII. Bound FXII was detected by subsequent incubation with ¹²⁵I-anti-FXII antibodies. As shown in Fig. 5, immobilized mAb KOK5 bound 100 times more FXII in DS-plasma than in EDTA-plasma thus indicating that mAb KOK5 bound to an epitope preferentially exposed on activated FXII. Immobilized mAb F3, studied as control, did not bind preferentially activated FXII (data not shown).

### 5.2.6 Identification of the epitope of mAb KOK5

To localize the epitope recognized by the mAb KOK5 we screened two phage-displayed random peptide libraries, a 15-mer peptide library and a 9-mer peptide library. After three rounds of affinity purification, 24 individually isolated phage clones from the 15-mer library were tested by ELISA for binding to mAb KOK5 or to an irrelevant mAb. Eighteen of the clones gave a positive signal in the ELISA test and were selected for further characterization. The amino acid sequences of the peptides displayed by the phages selected with mAb KOK5 were deduced from phage DNA sequence and are shown in table 2.

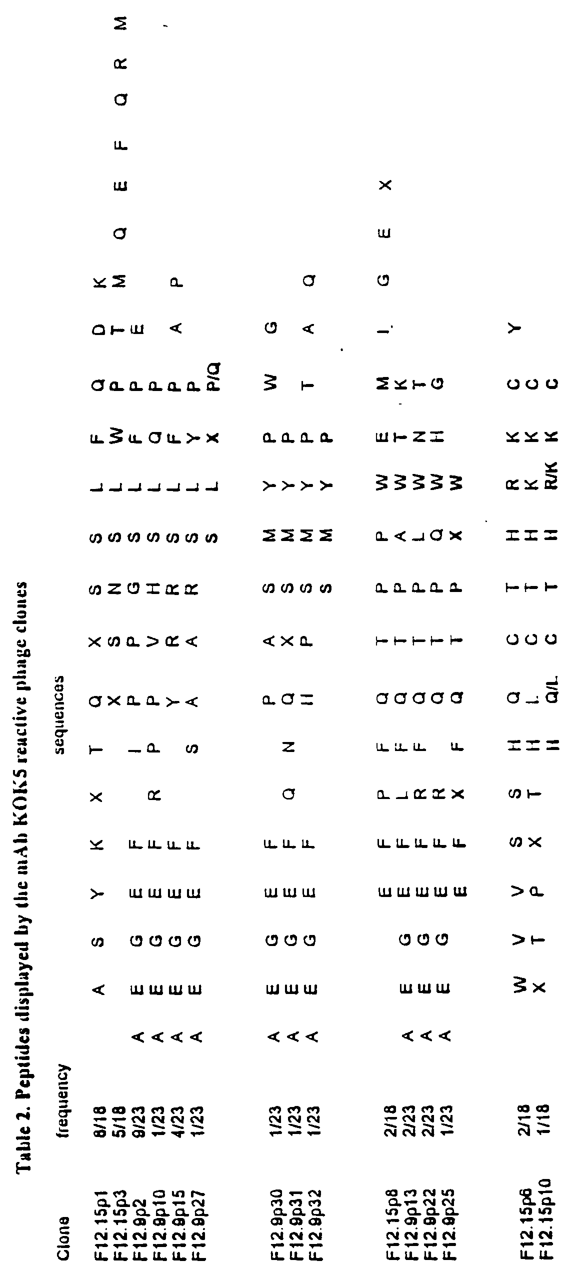

In table 2, deduced amino acid sequences of the 15-mer and 9-mer peptides displayed by phage clones isolated with mAb KOK5 as described in material and methods are shown. Sequences are given in the single letter code. The sequences have been aligned according to the most conserved amino acid residues and four consensus have been derived (bold). AEGEF represents the amino flanking region of the insertion in M13 PVIII protein (9-mer library) and has been used in some of the alignments. The FXII region in which alignments with the selected phage-displayed sequences was found, encompassed residues 28-60, i.e., CHFPFQYHRQLYHKCTHKGRPGPQPWCATTPNFDQDQR.

Comparison of the selected oligopeptides with one another allowed us to derive consensus sequences characterized by conserved amino acid residues. Thirteen clones, bearing two different sequences, showed the consensus SLXP/Q, were X was an amino acid with aromatic side chain, and was followed by a proline or a glutamine; implying either preferential affinity selection of these peptides, or over-representation of the phages that contained these peptides in the library. Three clones, harboring two different sequences (F12.15p6 and F12.15p10, in table 2), showed the consensus HQ/LCTHR/KKC. From the 9-mer peptide library 24 clones were isolated, 23 were found positive in the ELISA for binding to KOK5 and then sequenced. Fifteen clones, bearing 4 different sequences, showed the consensus sequence SLXP, similar to that found in the 15-mer library. Three clones, with a different sequence each, showed the consensus SMYP rather than SLXP. Finally, a sequence displayed by 5 independent clones could be aligned to the sequence of a clone (F12.15p8) isolated from the 15-mer library showing the consensus EFXFQTPXW.

### 5.2.7 Effects of synthetic peptides on binding of mAb KOK5 to FXII

To establish whether the peptides characterized as fusions with pIII or pVIII proteins of M13 phage would retain their immunological reactivity independently on the phage context, we chemically synthesized two peptides, AEGEFRFQTPQWE and SHQCTHRKCY, whose sequences corresponded to the consensus derived by aligning the two groups of isolated phages which displayed peptides with an homology with FXII primary sequence. In addition the two peptides: ²⁹HFPFQYHRQ³⁷ and ³⁹YHKCTHKGR⁴⁷, corresponding to the FXII sequences having maximal homology with the peptides diplayed by the isolated phages, were chemically synthesized. The 4 peptides were tested for: a) inhibition of binding between mAb KOK5 and FXII; b) inhibition of FXII activation by kallikrein in the presence of KOK5; c) inhibition of FXII binding to kaolin; d) inhibition of contact system activation by either DS500 or kaolin.

First the interaction of the synthetic peptides with mAb KOK5 by performing competitive ELISA was estimated. When we tested the capability of the different peptides to compete the binding of biotinylated mAb KOK5 to purified FXII immobilized on microplates we found that only the peptide AEGEFRFQTPQWE, corresponding to the sequence of one group of isolated phages, was able to compete, in a dose dependent manner, the binding of mAb KOK5 to FXII. The corresponding FXII peptide (residues 29-37), as well as the other two peptides, did not have any effect, at least at the concentrations used (Fig. 6). Consistently, the peptide AEGEFRFQTPQWE was able to inhibit FXII activation by kallikrein in the presence of mAb KOK5 (Fig. 7). The inhibition was dependent on the concentration of the synthetic peptide and the IC₅₀ was 50 µM. In the latter experiment a weak inhibition of FXII activation in the presence of the peptide ²⁹HFPFQYHRQ³⁷, harboring the primary sequence of FXII corresponding to that of the inhibiting peptide, was observed implying that also this peptide did bind, though with lower efficiency, to mAb KOK5 (Fig. 7).

### 5.2.8 Effects of synthetic peptides on binding of FXII to kaolin

Since mAb KOK5 inhibited the binding of FXII to kaolin we also studied whether the synthetic peptides were able to interfere with the binding of FXII with negatively charged surfaces. Peptides SHQCTHRKCY and ³⁹YHKCTHKGR⁴⁷, corresponding to the sequence of one group of isolated phages and the homologous FXII sequence, respectively, inhibited binding of ¹²⁵I-FXII to kaolin with an IC₅₀, 0.9 mM, 10,000 times higher than that of FXII itself. The peptide AEGEFRFQTPQWE did not show any effect on FXII binding to kaolin, neither did the corresponding FXII sequence (residues 29-37) (Fig. 8). This result prompted us to study the effects of the peptides SHQCTHRKCY and ³⁹YHKCTHKGR⁴⁷ on contact activation induced in EDTA-plasma by either DS500 or kaolin. In EDTA-plasma incubated with either DS500 or kaolin, an increasing amount of the synthetic peptides SHQCTHRKCY and ³⁹YHKCTHKGR⁴⁷ inhibited the activation of contact system as measured by the formation of FXIIa-C1-inhibitor complexes (Fig. 9). The concentration of ³⁹YHKCTHKGR⁴⁷ required to inhibit generation of FXIIa-C1-inhibitor complexes to 50% was 0.36 ± 0.13 mM in the presence of DS500 and 1 ± 0.28 mM in the presence of kaolin. Furthermore, ³⁹YHKCTHKGR⁴⁷ appeared to be at least ten times more efficient than SHQCTHRKCY (table 3). As well the peptides were able to inhibit to less than 50% the generation of the other protease-C1-inhibitor complexes studied (table 3). As expected from binding experiment results, FXII derived peptide 29-37 did not have any effect on contact system activation (Fig. 9).

**Table 3**

| Inhibition of contact system activation by synthetic peptides (IC₅₀ values) | | | | |
|---|---|---|---|---|
| | Activator | | | |
| | DS500 | DS500 | kaolin | kaolin |
| Synthetic peptides | phage-displayed peptide | FXII peptide | phage-displayed peptide | FXII peptide |
| FXIIa-C1inh | 3.15 ± 0.85 | 0.36 ± 0.13 | 7.00 ± 1.40 | 1.00 ± 0.28 |
| kal-C1inh | 5.25 ± 2.70 | 0.42 ± 0.07 | 6.00 ± 2.10 | 1.25 ± 0.75 |
| FXIa-C1inh | 6.75 ± 1.25 | 3.00 ± 1.00 | 7.50 ± 0.50 | 2.33 ± 0.62 |

20 µl of either DS500 (100 µg/ml) or kaolin (5 mg/ml) were incubated with 20 µl of serial dilutions of synthetic peptides (0.03-10 mM) and 40 µl of EDTA-plasma as described in material and methods. The inhibition displayed by the phage-displayed peptide (SHQCTHRKCY) and the FXII peptide (³⁹YHKCTHKGR⁴⁷) is expressed as IC₅₀ value (i.e. the concentration in mM of peptides that inhibited to 50% the generation of protease-C1-inh complexes).

### 6. Conclusions

The experiments described above indicate that:
- mAb KOK5 a) inhibits the binding of FXII to an activator; b) has no effect on the activity of FXII bound to an activator; and c) induces activation of FXII. Hence, mAb KOK5 is directed against the site on FXII that mediates binding of FXII to and activation of FXII by an activator.
- the residues 29-37 and 39-47 of the heavy chain region of factor XII contribute to the epitope for mAb KOK5.
- peptides mimicking the sequence of residues 39-47 of the heavy chain region of FXII (i.e., the sequence YHKCTHKGR) inhibit the binding of FXII to an activator and inhibit contact activation in plasma by an activator.
- the sequence ³⁹YHKCTHKGR⁴⁷ of the heavy chain region of FXII (in part) constitutes the binding site of FXII to an activator.

## Claims

1. A peptide capable of competing with Factor XII for binding to a Factor XII activator and inhibiting activation of the contact system of coagulation, wherein the peptide comprises the amino acid sequence ³⁹YHKCTHKGR⁴⁷ (SEQ ID NO: 1) or a functionally equivalent sequence.

2. A peptide according to claim 1, which comprises the fibronectin type II homology region of factor XII having the amino acid sequence: ¹³KAEEHTVVLTVTGEPCHFPFQYHRQLYHKCTHKGRPGPQPWCATTPNFDQDQRWGYC⁶⁹ (SEQ ID NO: 2), or a part thereof which includes SEQ ID NO: 1.

3. A peptide according to claim 2, which comprises the amino acid sequence: ¹⁶EHTVVLTVTGEPCHFPFQYHRQLYHKCTHKGRPGPQPWCATTPNFDQ DQRWGYC⁶⁹ (SEQ ID NO: 3).

4. A peptide according to claim 1, further comprising an amino acid sequence not derived from Factor XII, or a carrier protein, or both.

5. A pharmaceutical composition, comprising a peptide according to any one of claims 1-4 in an amount effective to inhibit activation of the contact system of coagulation and a pharmaceutically acceptable carrier or diluent.

6. A method for inhibiting activation of the contact system of coagulation by artificial surfaces to be exposed to blood, comprising treating said artificial surfaces with a peptide according to any one of claims 1-4.

7. A method of treatment of an animal, including a human, at risk for activation of the contact system of coagulation, comprising administering to said animal a peptide according to any one of claims 1-4 in an amount effective to inhibit said activation of the contact system of coagulation.

8. Use of a peptide according to any one of claims 1-4 for preparing a pharmaceutical composition for treating an animal, including a human, to inhibit activation of the contact system of coagulation.


SEQUENCE LISTING

(1) GENERAL INFORMATION:

(i) APPLICANT:
(A) NAME: Stichting Sanquin Bloedvoorziening
(B) STREET: Plesmanlaan 125
(C) CITY: Amsterdam
(D) STATE: Noord-Holland
(E) COUNTRY: the Netherlands
(F) POSTAL CODE (ZIP): 1066 CX

(ii) TITLE OF INVENTION: Inhibition of contact system activation

(iii) NUMBER OF SEQUENCES: 25

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98202181.8

(2) INFORMATION FOR SEQ ID NO: 1:

(i) SEQUENCE CHARACTERISTICS:
(A) LENGTH: 9 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 1:

Tyr His Lys Cys Thr His Lys Gly Arg
1               5

(2) INFORMATION FOR SEQ ID NO: 2:

(i) SEQUENCE CHARACTERISTICS:
(A) LENGTH: 57 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 2:

Lys Ala Glu Glu His Thr Val Val Leu Thr Val Thr Gly Glu Pro Cys
1               5                   10                  15

His Phe Pro Phe Gln Tyr His Arg Gln Leu Tyr His Lys Cys Thr His
            20                  25                  30

Lys Gly Arg Pro Gly Pro Gln Pro Trp Cys Ala Thr Thr Pro Asn Phe
        35                  40                  45

Asp Gln Asp Gln Arg Trp Gly Tyr Cys
    50                  55

(2) INFORMATION FOR SEQ ID NO: 3:

(i) SEQUENCE CHARACTERISTICS:
(A) LENGTH: 38 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 3:

Cys His Phe Pro Phe Gln Tyr His Arg Gln Leu Tyr His Lys Cys Thr
1               5                   10                  15

His Lys Gly Arg Pro Gly Pro Gln Pro Trp Cys Ala Thr Thr Pro Asn
            20                  25                  30

Phe Asp Gln Asp Gln Arg
        35

(2) INFORMATION FOR SEQ ID NO: 4:

(i) SEQUENCE CHARACTERISTICS:
(A) LENGTH: 13 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 4:

Ala Glu Gly Glu Phe Arg Phe Gln Thr Pro Gln Trp Glu
1               5                   10

(2) INFORMATION FOR SEQ ID NO: 5:

(i) SEQUENCE CHARACTERISTICS:
(A) LENGTH: 10 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 5:

Ser His Gln Cys Thr His Arg Lys Cys Tyr
1               5                   10

(2) INFORMATION FOR SEQ ID NO: 6:

(i) SEQUENCE CHARACTERISTICS:
(A) LENGTH: 9 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 6:

His Phe Pro Phe Gln Tyr His Arg Gln
1               5

(2) INFORMATION FOR SEQ ID NO: 7:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 7:

Ala Ser Tyr Lys Xaa Thr Gln Xaa Ser Ser Leu Phe Gln Asp Lys
1               5                   10                  15

(2) INFORMATION FOR SEQ ID NO: 8:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 8:

Xaa Ser Asn Ser Leu Trp Pro Thr Met Gln Glu Phe Gln Arg Met
1               5                   10                  15

(2) INFORMATION FOR SEQ ID NO: 9:

(i) SEQUENCE CHARACTERISTICS:
(A) LENGTH: 14 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 9:

Ala Glu Gly Glu Phe Ile Pro Pro Gly Ser Leu Phe Pro Glu
1               5                   10

(2) INFORMATION FOR SEQ ID NO: 10:

(i) SEQUENCE CHARACTERISTICS:
(A) LENGTH: 14 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 10:

Ala Glu Gly Glu Phe Arg Pro Pro Val His Ser Leu Gln Pro
1               5                   10

(2) INFORMATION FOR SEQ ID NO: 11:

(i) SEQUENCE CHARACTERISTICS:
(A) LENGTH: 14 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 11:

Ala Glu Gly Glu Phe Tyr Arg Arg Ser Leu Phe Pro Ala Pro
1               5                   10

(2) INFORMATION FOR SEQ ID NO: 12:

(i) SEQUENCE CHARACTERISTICS:
(A) LENGTH: 13 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 12:

Ala Glu Gly Glu Phe Ser Ala Ala Arg Ser Leu Tyr Pro
1               5                   10

(2) INFORMATION FOR SEQ ID NO: 13:

(i) SEQUENCE CHARACTERISTICS:
(A) LENGTH: 13 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 13:

Ala Glu Gly Glu Phe Pro Ala Ser Met Tyr Pro Trp Gly
1               5                   10

(2) INFORMATION FOR SEQ ID NO: 14:

(i) SEQUENCE CHARACTERISTICS:
(A) LENGTH: 13 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 14:

Ala Glu Gly Glu Phe Gln Asn Gln Xaa Ser Met Tyr Pro
1               5                   10

(2) INFORMATION FOR SEQ ID NO: 15:

(i) SEQUENCE CHARACTERISTICS:
(A) LENGTH: 14 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 15:

Ala Glu Gly Glu Phe His Pro Ser Met Tyr Pro Thr Ala Gln
1               5                   10

(2) INFORMATION FOR SEQ ID NO: 16:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 16:

Glu Phe Pro Phe Gln Thr Pro Pro Trp Glu Met Ile Gly Glu Xaa
1               5                   10                  15

(2) INFORMATION FOR SEQ ID NO: 17:

(i) SEQUENCE CHARACTERISTICS:
(A) LENGTH: 14 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 17:

Ala Glu Gly Glu Phe Leu Phe Gln Thr Pro Ala Trp Thr Lys
1               5                   10

(2) INFORMATION FOR SEQ ID NO: 18:

(i) SEQUENCE CHARACTERISTICS:
(A) LENGTH: 14 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 18:

Ala Glu Gly Glu Phe Arg Phe Gln Thr Pro Leu Trp Asn Thr
1               5                   10

(2) INFORMATION FOR SEQ ID NO: 19:

(i) SEQUENCE CHARACTERISTICS:
(A) LENGTH: 13 amino acids
(B) TYPE: amino acid

(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 19:

Ala Glu Gly Glu Phe Arg Gln Thr Pro Gln Trp His Gly
1               5                   10

(2) INFORMATION FOR SEQ ID NO: 20:

(i) SEQUENCE CHARACTERISTICS:
(A) LENGTH: 14 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 20:

Trp Val Val Ser Ser His Gln Cys Thr His Arg Lys Cys Tyr
1               5                   10

(2) INFORMATION FOR SEQ ID NO: 21:

(i) SEQUENCE CHARACTERISTICS:
(A) LENGTH: 13 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 21:

Xaa Thr Pro Xaa Thr His Leu Cys Thr His Lys Lys Cys
1               5                   10

(2) INFORMATION FOR SEQ ID NO: 22:

(i) SEQUENCE CHARACTERISTICS:

(A) LENGTH: 4 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(ix) FEATURE:
(A) NAME/KEY: Peptide
(B) LOCATION:3..4
(D) OTHER INFORMATION:/note= "Regarding position 4: "P" (Pro)
can be P or Q (Gln)."

(xi) SEQUENCE DESCRIPTION: SEQ ID NO: 22:

Ser Leu Xaa Pro
1

(2) INFORMATION FOR SEQ ID NO: 23:

(i) SEQUENCE CHARACTERISTICS:
(A) LENGTH: 4 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 23:

Ser Met Tyr Pro
1

(2) INFORMATION FOR SEQ ID NO: 24:

(i) SEQUENCE CHARACTERISTICS:
(A) LENGTH: 9 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 24:

Glu Phe Xaa Phe Gln Thr Pro Xaa Trp
1               5

(2) INFORMATION FOR SEQ ID NO: 25:

(i) SEQUENCE CHARACTERISTICS:
(A) LENGTH: 9 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(ix) FEATURE:
(A) NAME/KEY: Peptide
(B) LOCATION:2..6
(D) OTHER INFORMATION:/note= "Regarding position 2: "Q" (Gln)
can be Q or L (Leu). Regarding position 6: "R" (Arg) can be R or K (Lys)."

(xi) SEQUENCE DESCRIPTION: SEQ ID NO: 25:

His Gln Cys Thr His Arg Lys Cys
1               5